(19) Europäisches Patentamt
European Patent Office
Office européen des brevets

(11) **EP 1 212 962 B1**

(12) **FASCICULE DE BREVET EUROPEEN**

(45) Date de publication et mention
de la délivrance du brevet:
**31.01.2007 Bulletin 2007/05**

(51) Int Cl.:
***A45D 44/00*** *(2006.01)*

(21) Numéro de dépôt: **01403168.6**

(22) Date de dépôt: **07.12.2001**

(54) **Nuancier**

Farbschattierungssammlung

Shade chart

(84) Etats contractants désignés:
**AT BE CH CY DE DK ES FI FR GB GR IE IT LI LU MC NL PT SE TR**

(30) Priorité: **08.12.2000 FR 0016001**

(43) Date de publication de la demande:
**12.06.2002 Bulletin 2002/24**

(73) Titulaire: **L'ORÉAL**
**75008 Paris (FR)**

(72) Inventeurs:
• **De Rigal, Jean**
**Gressy,**
**77410 Claye Souilly (FR)**
• **Dauga, Christophe**
**92300 Levallois-Perret (FR)**

(74) Mandataire: **Tanty, François**
**Nony & Associés,**
**3, rue de Penthièvre**
**75008 Paris (FR)**

(56) Documents cités:
**FR-A- 2 540 991          US-A- 1 741 080**
**US-A- 5 311 293**

• **PATENT ABSTRACTS OF JAPAN vol. 017, no. 457 (P-1597), 20 août 1993 (1993-08-20) & JP 05 107115 A (KAZUMI TAWARA), 27 avril 1993 (1993-04-27)**
• **PATENT ABSTRACTS OF JAPAN vol. 1997, no. 09, 30 septembre 1997 (1997-09-30) & JP 09 133584 A (KAO CORP), 20 mai 1997 (1997-05-20)**

**Description**

La présente invention concerne un nuancier regroupant une pluralité de modèles de comparaison destinés chacun à reproduire l'apparence d'un élément kératinique tel que la peau, les lèvres, les ongles ou les cheveux.

On connaît par les brevets JP 05 107115, US 1 741 080 et US 5 311 293 des modèles de comparaison reproduisant la couleur de la peau, avec une variété de teintes.

Par le brevet FR-A-2 540 991 on connait un nuancier pour mesurer les degrés de coloration de surfaces, en particulier de la peau, par comparaison avec des nuances de coloration numérotées. Ce dispositif peut s'appliquer dans le domaine de la comparaison d'aspects de surface.

Il existe un besoin pour disposer d'un nuancier reproduisant fidèlement l'apparence de la peau, des lèvres, des ongles ou des cheveux.

La présente invention vise notamment à répondre à ce besoin.

Selon l'invention, le nuancier est défini par la revendication 1.

Le nuancier peut ainsi comporter au moins un modèle de comparaison reproduisant l'apparence d'un élément kératinique, notamment un élément kératinique tel que la peau, chaque modèle de comparaison reproduisant une couleur et une caractéristique d'apparence autre que la couleur.

Au moins deux modèles de comparaison reproduisent cette caractéristique d'apparence autre que la couleur à différents degrés.

Le nuancier selon l'invention peut reproduire ainsi non seulement différentes couleurs mais également au moins une caractéristique d'apparence autre que la couleur, à différents degrés, ce qui permet de faire des distinctions plus fines entre les différents types d'éléments kératiniques, distinctions faisant intervenir d'autres considérations que la couleur.

Dans une mise en oeuvre particulière de l'invention, la caractéristique d'apparence précitée est la brillance. De préférence, le nuancier comporte au moins deux modèles de comparaison ayant des brillances différentes. Ainsi, le nuancier permet de distinguer par exemple une peau mate d'une peau brillante, même si celles-ci sont de même couleur.

Le nuancier peut comporter au moins trois degrés de ladite caractéristique d'apparence autre que la couleur, voire au moins quatre, voire encore au moins cinq.

La caractéristique d'apparence autre que la couleur n'est pas limitée à la brillance. Au moins deux modèles de comparaison ait par exemple des couleurs réparties différemment au sein de chaque modèle de comparaison. Au moins deux modèles de comparaison peuvent par exemple comporter des taches d'une couleur différente de celle du fond, différentes en nombre et/ou en répartition et/ou en taille d'un modèle à l'autre. De telles taches peuvent être par exemple représentatives de celles qui apparaissent lorsque la peau vieillit, sous l'action du soleil notamment.

La caractéristique d'apparence autre que la couleur peut encore être par exemple l'hétérogénéité de relief, au moins deux modèles de comparaison ayant des distributions différentes du relief.

Les modèles de comparaison peuvent présenter une brillance non homogène, notamment lorsqu'il s'agit d'imiter l'aspect de la peau, en particulier son caractère localement plus ou moins brillant.

De préférence, pour obtenir une telle brillance non homogène, les modèles de comparaison comportent en surface une juxtaposition de zones élémentaires ayant des brillances différentes.

Les zones les plus brillantes peuvent devoir leur brillance à la présence d'un vernis brillant et les zones les moins brillantes peuvent devoir leur matité à la présence d'un vernis mat.

La largeur des zones les plus brillantes peut être voisine de 300 µm et celle des zones les moins brillantes voisine de 100 µm, lorsque les modèles de comparaison visent à imiter l'aspect de la peau.

Dans une mise en oeuvre particulière de l'invention, les modèles de comparaison comportent un relief déterminé leur conférant une brillance inhomogène.

Ce relief définit avantageusement des creux et des plateaux, les plateaux pouvant être recouverts d'un vernis brillant tandis que les creux en sont dépourvus.

En variante, chaque modèle de comparaison peut présenter une brillance homogène.

De préférence, chaque modèle de comparaison est réalisé au moyen de pigments et/ou de colorants choisis de telle sorte que son spectre de réflectance présente un profil suffisamment proche de celui de l'élément kératinique dont il reproduit la couleur pour que ce dernier et le modèle de comparaison correspondant apparaissent, pour un observateur, de la même couleur sous au moins deux illuminants différents.

Ainsi, un utilisateur du nuancier tel qu'une esthéticienne, une clinicienne ou un dermatologue, voire une personne sans qualification particulière en cosmétique, peut sélectionner sous un illuminant donné un modèle de comparaison, correspondant par exemple à une couleur de peau que l'on cherche à évaluer, tout en étant assuré que le résultat de la sélection reste valable sous un autre illuminant.

De préférence, chaque modèle de comparaison reproduit fidèlement la couleur de l'élément kératinique au moins sous deux des illuminants D65 (lumière du jour), D50 et A (lampe à incandescence).

Selon un aspect de l'invention, l'écart de couleur induit par le changement d'illuminant est inférieur à 4 et de préférence encore inférieur à 2.

L'évaluation de la couleur de la peau ou de la couleur d'un élément kératinique autre que la peau est ainsi facilitée, puisqu'il n'est pas nécessaire d'utiliser au moment de la comparaison une lampe ayant un spectre d'émission spécifique.

Le spectre de réflectance de chaque modèle de comparaison peut rester proche de celui de l'élément kératinique correspondant dans un domaine spectral s'étendant de préférence de 400 à 800 nm.

De préférence,

$$\text{la quantité } 1/N(\lambda) \sum_{\lambda} \left| I_R^{MOD}(\lambda) - I_R^{REF}(\lambda) \right| / I_R^{REF}(\lambda),$$

où $I_R^{MOD}(\lambda)$ est l'intensité lumineuse réfléchie à la longueur d'onde $\lambda$ pour le modèle de comparaison et $I_R^{REF}(\lambda)$ est l'intensité lumineuse réfléchie à la longueur d'onde $\lambda$ pour l'élément kératinique, est inférieure ou égal à 0,1 de préférence inférieure à 0,05 et de préférence encore < 0,01.

Dans le cas notamment où le nuancier comporte des modèles de comparaison reproduisant des couleurs de peau, les modèles de comparaison présentent avantageusement des teintes différentes, les angles de teinte étant de préférence compris entre 40° et 70°, et de préférence entre 46° et 64° dans l'espace colorimétrique CIEL*C*h 1976.

Le nuancier comporte par exemple au moins dix catégories de modèles de comparaison présentant chacune une teinte déterminée, différente de celle des autres catégories.

Les modèles de comparaison peuvent aussi avantageusement présenter des clartés différentes, les niveaux de clarté (L* dans l'espace colorimétrique CIEL*C*h 1976) étant de préférence compris entre 34 et 75.

Le nuancier comporte par exemple au moins cinq catégories de modèles de comparaison présentant chacune un niveau de clarté déterminé, différent de celui des autres catégories.

Dans une mise en oeuvre préférée de l'invention, le nuancier comporte cinquante couleurs correspondant à la combinaison de dix teintes et de cinq niveaux de clarté.

De préférence, l'écart de couleur global ΔE*C*h.94 mesuré dans l'espace colorimétrique CIEL*C*h 1976, entre deux modèles de comparaison correspondant à des couleurs de peau voisines, est constant, cet écart étant de préférence compris entre 1 et 40, de préférence entre 1 et 20, et de préférence encore voisin de 4.

Au sein d'un même modèle de comparaison, la couleur peut être uniforme et constante sur l'ensemble de la surface du modèle de comparaison ou être non uniforme, afin d'imiter la texture de la peau par exemple.

Un modèle de comparaison peut ainsi recevoir deux revêtements colorés de couleurs différentes, l'ensemble produisant une couleur moyenne pour l'oeil.

Un modèle de comparaison peut aussi recevoir un revêtement coloré sur un support non lisse dont le relief est choisi de manière à imiter le grain de la peau.

Les modèles de comparaison peuvent être réalisés chacun sur un support de forme générale rectangulaire et de dimensions voisines de 60 mm par 100 mm par exemple.

Le nuancier peut comporter plusieurs modèles de comparaison reliés en éventail.

Le nuancier peut également comporter plusieurs modèles de comparaison sur un même support, de préférence en forme de bande.

Les modèles de comparaison sont avantageusement chacun réalisés avec un support comportant un trou, situé par exemple à environ un tiers de leur longueur, ce trou étant par exemple circulaire de diamètre voisin de 20 mm.

Chaque modèle de comparaison peut comporter un identifiant propre, tel qu'un code alphanumérique, de manière à permettre à l'utilisateur d'identifier aisément les différents modèles de comparaison.

Chaque modèle de comparaison peut comporter un identifiant associé à la couleur et à ladite au moins une caractéristique d'apparence autre que la couleur du modèle. Un tel identifiant peut être un code alphanumérique.

Les modèles de comparaison peuvent être configurés pour simuler l'apparence d'un élément kératinique choisi parmi les cheveux, la peau, les ongles des mains et des pieds.

Chaque modèle de comparaison peut être configuré de manière à être affiché sur un emballage d'un produit destiné à être appliqué sur l'élément kératinique.

L'invention a encore pour objet, selon un autre de ses aspects, un procédé pour fabriquer un produit destiné à une application sur un élément kératinique, ce procédé comportant les étapes suivantes :

- fournir un nuancier tel que défini plus haut,
- sélectionner au moins l'un d'une pluralité de modèles de comparaison, et
- fabriquer un produit destiné à l'application sur un élément kératinique en fonction de la couleur et de la caractéristique d'apparence autre que la couleur dudit au moins un modèle de comparaison sélectionné.

La sélection dudit au moins un modèle de comparaison peut comporter la détermination duquel de la pluralité de modèles de comparaison correspond sensiblement à une couleur et à une caractéristique d'apparence autre que la couleur de l'élément kératinique sur lequel le produit est destiné à être appliqué.

La sélection dudit au moins un modèle de comparaison peut comporter la détermination duquel des modèles de comparaison correspond sensiblement à une couleur et à une caractéristique d'apparence autre que la couleur, souhaitées par l'utilisateur du produit, ce produit pouvant être un produit cosmétique ou de soins, par exemple un produit tel qu'un fond de teint, un produit pour masquer les imperfections de la peau, un produit pour les lèvres, un colorant capillaire, un produit pour le soin des cheveux, un vernis à ongles, un blush, une ombre à paupières, un produit de coloration de la peau ou un produit pour le soin de la peau.

L'invention a encore pour objet un procédé pour suivre le traitement cosmétique, d'un élément kératinique avec un produit, ce procédé comportant les étapes suivantes :

- fournir le nuancier tel que précité,
- sélectionner un modèle de comparaison qui correspond sensiblement à une couleur et à au moins une caractéristique d'apparence autre que la couleur de l'élément kératinique,
- appliquer le produit sur l'élément kératinique, et
- déterminer si la couleur et/ou ladite au moins une caractéristique d'apparence autre que la couleur de l'élément kératinique sur lequel le produit a été appliqué a été modifiée après avoir appliqué le produit, en comparant l'élément kératinique et les modèles de comparaison.

Ce procédé peut comporter la sélection parmi une pluralité de produits différents du produit à appliquer sur l'élément kératinique, cette sélection étant fonction du modèle de comparaison sélectionné. Chacun des modèles de comparaison peut comporter un identifiant associé à la couleur et à ladite au moins une caractéristique d'apparence autre que la couleur du modèle, et les différents produits peuvent comporter des identifiants qui correspondent respectivement aux identifiants des modèles de comparaison, le produit sélectionné comportant le même identifiant que le modèle de comparaison sélectionné.

Le produit peut affecter le relief de l'élément kératinique ou sa couleur.

L'invention a encore pour objet, selon un autre de ses aspects, un procédé pour sélectionner un produit cosmétique, destiné à être appliqué sur un élément kératinique, le procédé comportant les étapes suivantes :

- fournir le nuancier tel que précité,
- sélectionner un modèle de comparaison ayant une couleur et au moins une caractéristique d'apparence autre que la couleur qui correspond sensiblement à l'élément kératinique sur lequel le produit est destiné à être appliqué, et
- sélectionner un produit d'une pluralité de produits différents destiné à être appliqués sur l'élément kératinique, en fonction du modèle de comparaison sélectionné.

L'invention a encore pour objet, selon un autre de ses aspects, un procédé de traitement cosmétique d'un élément kératinique, ce procédé comportant les étapes suivantes :

- fournir le nuancier précité,
- sélectionner un modèle de comparaison dudit nuancier qui correspond à une couleur désirée et à au moins une caractéristique d'apparence désirée, autre que la couleur de l'élément kératinique,
- traiter l'élément kératinique en fonction du modèle de comparaison sélectionné.

Le procédé peut comporter l'association de chacun des modèles de comparaison avec l'emballage d'un produit.

Dans le procédé pour réaliser un nuancier, on peut regrouper une pluralité de modèles de comparaison reproduisant chacun la couleur d'un élément kératinique, notamment un élément kératinique tel que la peau, et une caractéristique d'apparence autre que la couleur, notamment la brillance.

L'un au moins du support et du revêtement peut subir un traitement destiné à lui permettre d'imiter la texture de la peau par exemple.

Un tel traitement peut comporter un embossage du support, voire du revêtement.

Le traitement peut également consister à imprimer des motifs sur un fond coloré, les motifs ayant une couleur différente de celle du fond.

Le traitement peut encore consister à imprimer un vernis mat ou brillant, afin de conférer une brillance hétérogène au modèle de comparaison.

Le cas échéant, le modèle de comparaison peut recevoir une impression d'un vernis mat et une impression d'un vernis brillant.

Le choix des pigments et/ou colorants utilisés pour réaliser le revêtement pourra être effectué en fonction de l'incidence sur la couleur finale du traitement mis en oeuvre.

L'invention a encore pour objet un procédé de fabrication d'un produit cosmétique, notamment un fond de teint, caractérisé par le fait qu'il comprend les étapes suivantes :

- fournir un nuancier tel que défini plus haut,
- déterminer le modèle de comparaison correspondant à la couleur et à la caractéristique d'apparence autre que la couleur de la peau d'une personne ou correspondant à une couleur et une caractéristique d'apparence autre que la couleur souhaitée,
- fabriquer un produit cosmétique, notamment un fond de teint, ayant la couleur et la caractéristique d'apparence autre que la couleur du modèle de comparaison ainsi déterminé, en vue de son application sur la peau de cette personne.

Dans le procédé ci-dessus, le nuancier peut être fourni matériellement.

L'invention a encore pour objet un procédé pour déterminer l'efficacité d'un produit cosmétique notamment un produit ayant une action sur la brillance de la peau, caractérisé par le fait qu'il comporte les étapes suivantes :

- déterminer parmi les modèles de comparaison du nuancier tel que défini plus haut, celui dont la couleur et la caractéristique d'apparence autre que la couleur, notamment la brillance, sont les plus proches de celles de la peau d'une personne donnée,
- appliquer sur la peau de cette personne ou administrer le produit cosmétique ou de soin,
- rechercher une éventuelle variation de la couleur et/ou de la caractéristique d'apparence, notamment la brillance de la peau, en procédant à une nouvelle comparaison avec les modèles de comparaison du nuancier.

Un tel procédé peut permettre de quantifier aisément la variation de couleur et/ou de brillance ou la variation d'une autre caractéristique d'apparence autre que la couleur de la peau suite à un traitement donné, donc d'évaluer l'efficacité de celui-ci.

Le nuancier tel que défini plus haut peut être utilisé pour déterminer l'état de santé d'un individu, en détectant à l'aide du nuancier une variation éventuelle anormale de la couleur ou d'une caractéristique d'apparence autre que la couleur, notamment une variation de la brillance, de la peau de cet individu.

L'invention a encore pour objet un procédé de sélection d'un produit cosmétique caractérisé par le fait qu'il comporte les étapes suivantes :

- faire figurer sur chaque emballage d'une même gamme de produits cosmétiques ayant différentes couleurs et différentes brillances des identifiants tels qu'un code alphanumérique spécifique à la couleur et à la brillance de chaque produit,
- faire figurer les mêmes identifiants sur les modèles de comparaison du nuancier tel que défini plus haut,
- déterminer parmi les modèles de comparaison du nuancier celui dont la couleur et la brillance sont les plus proches de celles de la peau d'un individu donné,
- sélectionner, en vue de l'application sur la peau de cet individu, le produit dont l'emballage comporte le même identifiant que celui figurant sur le modèle de comparaison précédemment déterminé.

L'invention a encore pour objet un procédé de traitement cosmétique, caractérisé par le fait qu'il comporte les étapes suivantes :

- fournir un nuancier tel que défini plus haut,
- déterminer la couleur et une caractéristique d'apparence autre que la couleur d'un élément kératinique à l'aide dudit nuancier,
- effectuer un traitement cosmétique en fonction de la couleur et de la caractéristique d'apparence précédemment déterminées.

On entend par traitement cosmétique tout traitement non thérapeutique au moyen d'un produit cosmétique tel que défini dans la Directive 76/768/CEE modifiée par la Directive 93/35/CEE du Conseil du 14 juin 1993, un maquillage étant un exemple de traitement cosmétique.

D'autres caractéristiques et avantages de la présente invention ressortiront à la lecture de la description détaillée qui va suivre, d'exemples de mise en oeuvre non limitatifs, et à l'examen du dessin annexé, sur lequel :

- la figure 1 représente schématiquement un nuancier conforme à un premier exemple de mise en oeuvre de l'invention,
- les figures 2 et 3 illustrent schématiquement deux variantes de mise en oeuvre de l'invention,

- la figure 4 illustre l'utilisation d'un modèle de comparaison,
- la figure 5 représente le spectre de réflectance d'une peau et celui du modèle de comparaison correspondant,
- les figures 6 à 9 illustrent schématiquement différentes structures de revêtement ou de support permettant de restituer une brillance hétérogène, et
- la figure 10 représente de manière très schématique un système permettant l'impression à distance d'un modèle de comparaison.

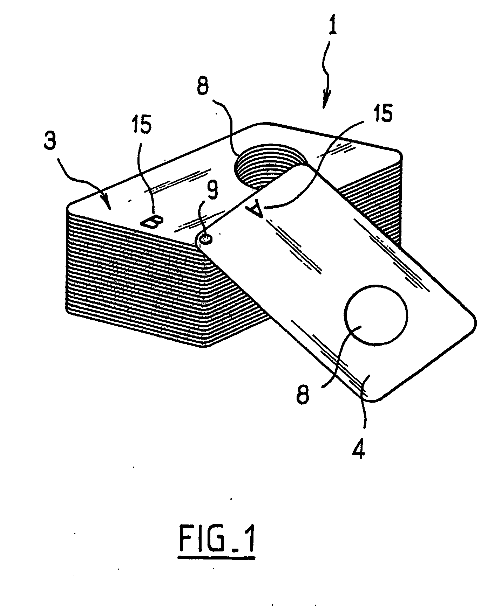

On a représenté sur la figure 1 un nuancier 1 conforme à l'invention, ce nuancier comportant plusieurs modèles de comparaison 4 reproduisant chacun une couleur de peau.

Chaque modèle de comparaison 4 comporte, dans l'exemple décrit, un support recouvert d'un revêtement coloré.

Chaque modèle de comparaison présente une forme sensiblement rectangulaire, de dimensions voisines de 60 mm par 100 mm dans l'exemple décrit.

Chaque modèle de comparaison 4 est traversé par un trou 8, ici circulaire de diamètre égal à 20 mm environ et situé, dans l'exemple décrit, à environ un tiers de sa longueur.

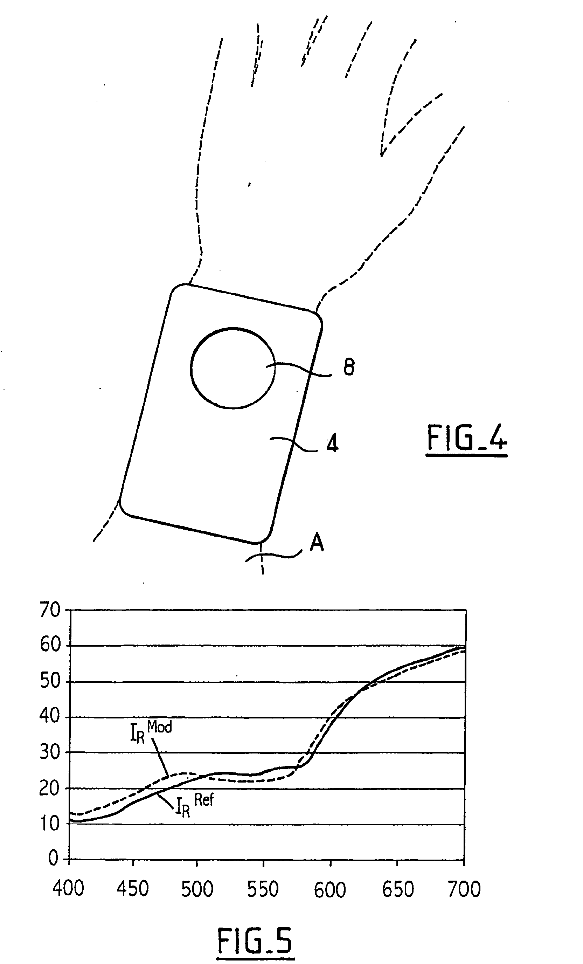

Un tel trou 8 permet, lorsqu'un modèle de comparaison 4 est posé sur une partie du corps ou du visage, par exemple l'avant-bras A comme illustré sur la figure 4, d'observer simultanément l'apparence de la peau et celle du modèle de comparaison 4, ce qui facilite la comparaison.

La forme rectangulaire du modèle de comparaison 4 permet éventuellement, par rapport à une forme carrée ou circulaire, d'orienter le modèle de comparaison 4 dans une direction particulière au moment de l'utilisation, ce qui s'avère préférable si le modèle de comparaison imite la texture de la peau, laquelle est anisotropique.

Chaque modèle de comparaison 4 comporte un identifiant 15 constitué par exemple par un ou plusieurs caractères alphanumériques.

Dans l'exemple décrit, le nuancier 1 comporte cent modèles de comparaison 4 correspondant à cinquante couleurs de peau différentes et deux brillances différentes par modèle, afin de distinguer une peau mate d'une peau brillante.

Tous les modèles de comparaison 4 présentent ici la même texture.

Les cinquante couleurs résultent, dans l'exemple décrit, de la combinaison de dix teintes et de cinq niveaux de clarté.

Les angles de teinte (h), mesurés dans l'espace CIEL*C*h 1976, sont compris entre 40 et 70, préférentiellement entre 46 et 64.

Les niveaux de clarté L* dans l'espace CIEL*C*h 1976 sont compris dans l'exemple décrit entre 25 et 80, préférentiellement entre 30 et 70.

Les niveaux de chroma sont préférentiellement compris entre 12 et 30, et de préférence autour de 22.

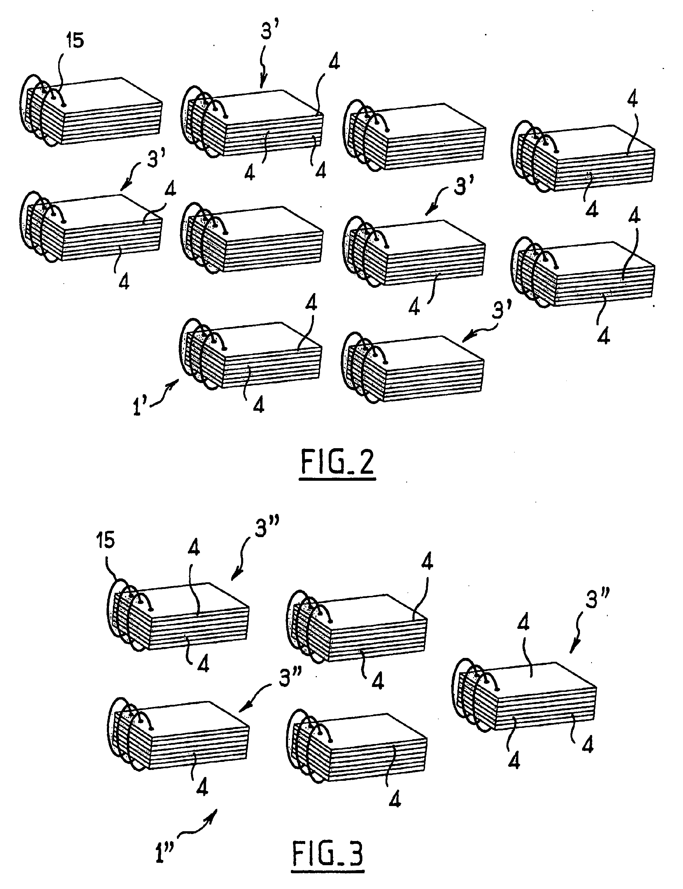

Les modèles de comparaison 4 peuvent être reliés en éventail en étant articulés autour d'un axe 9 comme cela est représenté sur la figure 1 ou être reliés au moyen de spires 15, comme cela est représenté sur les figures 2 et 3.

Par ailleurs, les modèles de comparaison 4 du nuancier peuvent être regroupés de plusieurs façons.

Tous les modèles de comparaison 4 peuvent être reliés ensemble au sein d'une liasse 3 unique, comme représenté sur la figure 1.

On peut encore constituer plusieurs groupes indépendants, afin de faciliter la manipulation des modèles de comparaison.

Le nuancier 1' représenté à la figure 2 comporte dix liasses 3' correspondant chacune à une teinte déterminée, différente de celle des autres liasses, chaque liasse 3' comportant deux ensembles de chacun cinq modèles de comparaison 4.

Au sein de chaque ensemble, les modèles de comparaison 4 présentent des niveaux de clarté différents mais une même teinte.

La brillance des modèles de comparaison d'un ensemble est différente de la brillance des modèles de comparaison de l'autre ensemble.

Le nuancier 1" représenté à la figure 3 comprend cinq liasses 3" correspondant chacune à un niveau de clarté déterminé, différent de celui des autres liasses, chaque liasse 3" comportant vingt modèles de comparaison 4 de teintes différentes mais de même clarté.

Une même liasse comporte des modèles de comparaison ayant une même teinte mais des brillances différentes.

D'autres regroupements peuvent encore être effectués sans que l'on sorte du cadre de la présente invention.

L'écart de couleur visuel mesuré dans l'espace colorimétrique CIEL*C*h 1976 entre deux modèles de comparaison 4 de couleurs voisines est de préférence constant, par exemple égal à 4, cette valeur permettant à une personne non entraînée de percevoir aisément une variation de couleur entre deux modèles de comparaison 4.

Les modèles de comparaison 4 permettent de reproduire fidèlement la couleur de la peau quelque soit l'illuminant, notamment que ce soit la lumière du jour ou un éclairage artificiel de type à incandescence ou fluorescence.

D'une manière générale, on cherche à faire correspondre au mieux le spectre de réflectance du modèle de comparaison avec celui de la peau correspondante, dans la gamme de longueurs d'onde 400-800 nm.

Les modèles de comparaison 4 sont ainsi fabriqués à partir de la connaissance des spectres de réflectance de toutes les variétés de peaux caucasiennes, noires ou asiatiques notamment.

A titre d'exemple, on a représenté en trait plein sur la figure 5 l'intensité relative réfléchie $I_R^{REF}$ (en %) en fonction de la longueur d'onde (en nm) pour une peau donnée et, en trait interrompu, l'intensité relative réfléchie $I_R^{MOD}$ (en %) en fonction de la longueur d'onde (en nm) pour le modèle de comparaison correspondant.

De préférence, la quantité $1/N(\lambda) \sum_{\lambda} |I_R^{MOD} - I_R^{REF}|/I_R^{REF} \leq 0,1$ préférentiellement $\leq 0,05$ et de préférence encore $\leq 0,01$.

Il est possible de privilégier des sous-intervalles spectraux dans lesquels les spectres de réflectance du modèle de comparaison 4 et de la peau correspondante sont beaucoup plus proches, c'est-à-dire $\Delta$ plus petit.

On peut ainsi privilégier, par exemple, l'intervalle spectral [600 nm ; 750 nm] correspondant à la couleur rouge et à ses nuances.

On peut avoir, par exemple, $\Delta \leq 0,01$ dans cet intervalle spectral.

Pour obtenir le spectre de réflectance recherché pour chaque modèle de comparaison 4, on peut se servir de logiciels connus permettant de déterminer une composition pigmentaire correspondant à un spectre de réflectance donné.

On peut par exemple utiliser le logiciel DATAMATCH de la société DATACOLOR INTERNATIONAL.

La couleur des modèles de comparaison 4 peut être homogène.

En variante, on peut réaliser des modèles de comparaison 4 présentant chacun une couleur hétérogène, c'est-à-dire présentant des variations locales de teinte ou de clarté.

La réflectance spectrale du modèle de comparaison 4 correspond alors à une valeur moyenne, pour une surface de 1 cm de diamètre, par exemple.

Des motifs peuvent être réalisés sur les modèles de comparaison 4 afin d'imiter la texture de la peau.

Chaque modèle de comparaison 4 peut ainsi comporter des zones plus sombres et des zones plus claires, les zones sombres étant par exemple obtenues par impression de motifs avec une couleur différente de celle du fond, par exemple une couleur bistre.

Il est également possible d'imiter l'aspect de la peau en utilisant un support présentant un relief, par exemple un support embossé de manière à imiter le grain de la peau.

L'embossage peut être réalisé par exemple par calandrage, avant ou après le dépôt du revêtement coloré.

Pour mieux imiter l'apparence de la peau, et notamment son caractère localement plus ou moins brillant, il est souhaitable de conférer aux modèles de comparaison 4 une brillance non homogène.

Il est possible d'obtenir une brillance non homogène de plusieurs manières.

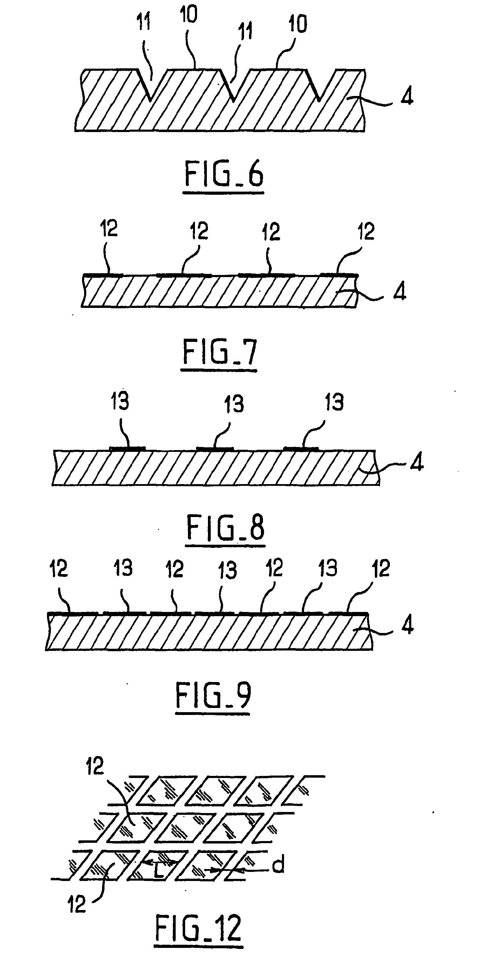

On peut notamment réaliser sur le support des plateaux 10 et des creux 11, comme cela est représenté sur la figure 6.

Les zones du revêtement coloré qui épousent la forme des creux 11 apparaissent alors moins brillantes que celles recouvrant les plateaux 10.

En variante, ou additionnellement, on peut appliquer sur les plateaux 10 un vernis brillant, les creux 11 en étant dépourvus.

On peut encore obtenir une brillance non homogène en appliquant sur un support plan, comme cela est illustré sur la figure 7, un vernis brillant 12 sur une partie seulement de la surface du modèle de comparaison, par exemple sous la forme de zones de 300 $\mu$m de largeur environ, espacées entre elles d'une distance de 100 $\mu$m environ.

En variante, comme cela est illustré sur la figure 8, on peut appliquer un vernis mat 13, par exemple sous la forme de zones de 100 $\mu$m de largeur, espacées entre elles d'une distance de 300 $\mu$m environ.

On peut encore juxtaposer ou superposer un vernis brillant et un vernis mat, les zones 12 de vernis brillant ayant une largeur voisine de 300 $\mu$m et les zones 13 de vernis mat ayant une largeur voisine de 100 $\mu$m, comme cela est représenté sur la figure 9.

Un utilisateur du nuancier détermine non seulement la couleur de la peau mais également la brillance correspondant à celle-ci.

Le nuancier peut trouver de nombreuses utilisations, dans le domaine de la cosmétique notamment.

Tout d'abord, le nuancier est utile pour effectuer des études statistiques dans la population, afin d'en extraire des caractéristiques typologiques par exemple.

Par ailleurs, le nuancier permet à une personne de connaître précisément la couleur et la brillance de sa peau, ce qui peut faciliter ensuite l'achat d'un produit cosmétique, notamment un fond de teint, cette personne n'ayant qu'à choisir le fond de teint affecté du même identifiant que celui présent sur le modèle de comparaison pour être sûre d'avoir la bonne couleur et la bonne brillance.

Le nuancier selon l'invention est également utile pour déterminer une variation de la couleur et/ou de la brillance de la peau, suite au traitement par un produit ou tout simplement suite à l'exposition au soleil ou à une source artificielle de rayonnement ultraviolet.

Le nuancier peut permettre de déterminer si le degré de bronzage souhaité est atteint pour une personne donnée.

Dans l'affirmative, cette personne est informée qu'une exposition ultérieure ne sera pas nécessaire, ce qui permet d'éviter une exposition excessive ou d'adopter une protection solaire.

Le nuancier peut également être utilisé pour déterminer l'effet sur la couleur et/ou la brillance de la peau d'un produit cosmétique ou de soin, par exemple un produit autobronzant.

Dans ce cas, l'utilisateur peut déterminer l'efficacité du traitement par comparaison avec un modèle de comparaison servant de référence et correspondant à la couleur et à la brillance de la peau avant traitement.

Le nuancier peut être proposé sous une forme matérielle telle que celle représentée sur les figures 1 à 3.

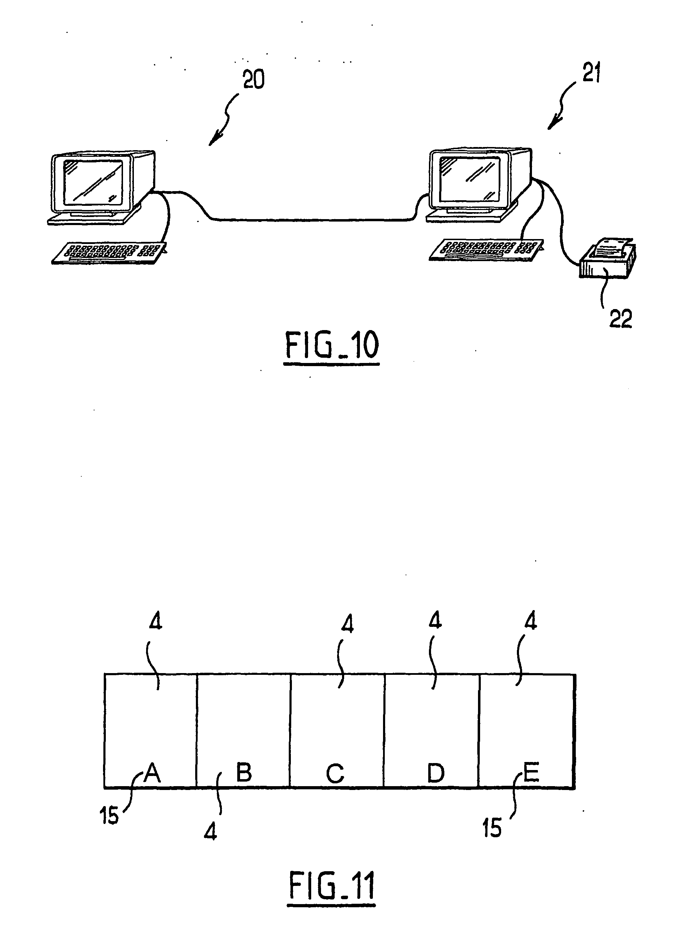

On a illustré sur la figure 10 le transfert depuis un premier ordinateur 20 vers un second ordinateur 21 d'un fichier de données informatiques contenant les informations nécessaires pour l'impression, au moyen d'une imprimante adaptée 22, des différents modèles de comparaison.

La transmission des données entre les ordinateurs 20 et 21 peut s'effectuer par exemple par un réseau informatique tel qu'Internet.

Bien sûr, l'invention n'est pas limitée aux exemples de mise en oeuvre qui viennent d'être décrits.

On peut notamment réaliser des nuanciers reproduisant fidèlement la couleur et une caractéristique d'apparence autre que la couleur, notamment la brillance, d'une lèvre, d'un ongle ou de cheveux d'un type déterminé.

La caractéristique d'apparence autre que la couleur peut résider dans le relief, ou l'hétérogénéité de relief.

Dans le cas du relief, les modèles de comparaison peuvent présenter plusieurs reliefs destinés à matérialiser par exemple les différents grains de peau.

De tels modèles de comparaison peuvent être utiles notamment pour déterminer l'action d'un produit ayant une influence sur le grain de la peau, notamment un produit ayant une action desquamante.

Lorsque la caractéristique d'apparence autre que la couleur est l'hétérogénéité de relief, les modèles de comparaison peuvent présenter un relief qui varie, selon plusieurs degrés, d'un relief homogène à un relief très irrégulier lors du vieillissement afin de matérialiser par exemple la manière dont le relief de la peau se modifie.

## Revendications

1. Nuancier, **caractérisé par le fait qu'**il comprend au moins deux modèles de comparaison configurés pour simuler l'apparence d'un élément kératinique choisi parmi la peau, les lèvres, les ongles et les cheveux, nuancier dans lequel lesdits au moins deux modèles de comparaison sont configurés pour simuler une couleur définie dans l'espace colorimétrique CIE L\*C\*h 1976, et à différents degrés au moins une caractéristique d'apparence autre que la couleur de l'élément kératinique.

2. Nuancier selon la revendication précédente, **caractérisé par le fait que** chaque modèle de comparaison (4) reproduit une couleur et une caractéristique d'apparence autre que la couleur.

3. Nuancier selon la revendication 1 ou 2, **caractérisé par le fait que** la caractéristique d'apparence autre que la couleur est la brillance, au moins deux modèles de comparaison (4) ayant de préférence des brillances différentes.

4. Nuancier selon la revendication 1 ou 2, **caractérisé par le fait que** la caractéristique d'apparence autre que la couleur est le relief, au moins deux modèles de comparaison ayant de préférence des reliefs différents.

5. Nuancier selon la revendication précédente, **caractérisé par le fait qu'**il comporte au moins deux modèles de comparaison ayant des taches d'une couleur différente de celle du fond, différentes en nombre et/ou en répartition et/ou en taille d'un modèle à l'autre.

6. Nuancier selon la revendication 1 ou 2, **caractérisé par le fait que** la caractéristique d'apparence autre que la couleur est l'hétérogénéité de relief, au moins deux modèles de comparaison ayant des distributions différentes du relief.

7. Nuancier selon l'une des revendications précédentes, **caractérisé par le fait qu'**il comporte au moins trois degrés de ladite caractéristique d'apparence autre que la couleur, voire au moins quatre, voire encore au moins cinq.

8. Nuancier selon l'une quelconque des revendications précédentes, **caractérisé par le fait que** les modèles de comparaison présentent une brillance non homogène.

9. Nuancier selon la revendication précédente, **caractérisé par le fait que** les zones les plus brillantes doivent leur brillance à la présence d'un vernis brillant (12).

10. Nuancier selon la revendication précédente, **caractérisé par le fait que** la largeur (L) des zones les plus brillantes est voisine de 300 $\mu$m.

11. Nuancier selon l'une des trois revendications immédiatement précédentes, **caractérisé par le fait que** les zones les moins brillantes doivent leur matité à la présence d'un vernis mat (13).

12. Nuancier selon la revendication précédente, **caractérisé par le fait que** la largeur (d) des zones les moins brillantes est voisine de 100 $\mu$m.

13. Nuancier selon l'une quelconque des revendications précédentes, **caractérisé par le fait que** les modèles de comparaison (4) comportent un relief déterminé leur conférant une brillance inhomogène.

14. Nuancier selon la revendication précédente, **caractérisé par le fait que** le relief est constitué de creux et de plateaux.

15. Nuancier selon la revendication précédente, **caractérisé par le fait que** les plateaux sont recouverts d'un vernis brillant, les creux en étant dépourvus.

16. Nuancier selon l'une quelconque des revendications 1 à 7, **caractérisé par le fait que** les modèles-de comparaison présentent une brillance homogène.

17. Nuancier selon l'une quelconque des revendications précédentes, **caractérisé par le fait que** les modèles de comparaison (4) sont chacun réalisés sur un support de forme générale rectangulaire et de dimensions voisines de 60 mm par 100 mm de préférence.

18. Nuancier selon l'une quelconque des revendications précédentes,
**caractérisé par le fait que** les modèles de comparaison (4) sont chacun réalisés sur un support comportant un trou (8), situé de préférence à environ un tiers de leur longueur, ce trou étant de préférence circulaire de diamètre voisin de 20 mm.

19. Nuancier selon l'une quelconque des revendications précédentes, **caractérisé par le fait que** chaque modèle de comparaison comporte un identifiant propre tel qu'un code alphanumérique.

20. Procédé de fabrication d'un produit cosmétique, notamment un fond de teint, **caractérisé par le fait qu'**il comprend les étapes suivantes :

  - fournir un nuancier selon l'une des revendications 1 à 19,
  - déterminer le modèle de comparaison correspondant à la couleur et à la caractéristique d'apparence autre que la couleur de la peau d'une personne ou correspondant à une couleur et une caractéristique d'apparence autre que la couleur souhaitée,
  - fabriquer un produit cosmétique notamment un fond de teint, ayant la couleur et la caractéristique d'apparence autre que la couleur du modèle de comparaison ainsi déterminé, en vue de son application sur la peau de cette personne.

21. Procédé pour déterminer l'efficacité d'un produit cosmétique notamment un produit ayant une action sur la brillance de la peau, **caractérisé par le fait qu'**il comporte les étapes suivantes :

  - déterminer parmi les modèles de comparaison du nuancier selon l'une des revendications 1 à 19 celui dont la couleur et la caractéristique d'apparence autre que la couleur, notamment la brillance, sont les plus proches de celles de la peau d'une personne donnée,
  - appliquer sur la peau de cette personne le produit,
  - rechercher une éventuelle variation de la couleur et/ou de la caractéristique d'apparence autre que la couleur, notamment la brillance, de la peau en procédant à une nouvelle comparaison avec les modèles de comparaison du nuancier.

22. Procédé pour sélectionner un produit destiné à être appliqué sur un élément kératinique, **caractérisé par le fait qu'**il comporte les étapes suivantes :

- fournir un nuancier tel que défini dans l'une quelconque des revendications 1 à 19,
- sélectionner un modèle de comparaison ayant une couleur et au moins une caractéristique d'apparence autre que la couleur qui correspond sensiblement à l'élément kératinique sur lequel le produit est destiné à être appliqué, et
- sélectionner un produit parmi une pluralité de produits différents destinés à être appliqués sur l'élément kératinique, en fonction du modèle de comparaison sélectionné.

23. Procédé de traitement cosmétique d'un élément kératinique, **caractérisé par le fait qu'**il comporte les étapes suivantes :

- fournir un nuancier tel que défini dans l'une quelconque des revendications 1 à 19,
- sélectionner un modèle de comparaison qui correspond à une couleur désirée et à au moins une caractéristique d'apparence désirée, autre que la couleur de l'élément kératinique,
- traiter l'élément kératinique en fonction du modèle de comparaison sélectionné.

24. Procédé pour suivre le traitement cosmétique, d'un élément kératinique avec un produit, **caractérisé par le fait qu'**il comporte les étapes suivantes :

- fournir un nuancier tel que défini dans l'une quelconque des revendications 1 à 19,
- sélectionner un modèle de comparaison qui correspond sensiblement à une couleur et à au moins une caractéristique d'apparence autre que la couleur de l'élément kératinique,
- appliquer le produit sur l'élément kératinique, et
- déterminer si la couleur et/ou ladite au moins une caractéristique d'apparence autre que la couleur de l'élément kératinique sur lequel le produit a été appliqué a été modifiée après avoir appliqué le produit, en comparant l'élément kératinique et les modèles de comparaison du système.

## Claims

1. A matching chart comprising at least two comparison samples configured to simulate the appearance of a keratinous element selected from the skin, the lips, the nails, and the hair **characterized by** the fact that the at least two comparison samples are configured to simulate a color defined in the CEE L*C*H 1976 color space and with different degrees at least one appearance characteristic other than color of the keratinous element.

2. A chart according to claim 1, **characterized by** the fact that each comparison sample (4) reproduces a color and an appearance characteristic other than color.

3. A chart according to claim 1 or 2, **characterized by** the fact that the appearance characteristic other than color is brightness, with at least two comparison samples (4) preferably having different brightnesses.

4. A chart according to claim 1 or 2, **characterized by** the fact that the appearance characteristic other than color is relief, with at least two comparison samples preferably having different amounts of relief.

5. A chart according to precedent claim, **characterized by** the fact that it includes at least two comparison samples having marks of a color different from a background color, the marks being different in number and/or distribution and/or size from one sample to another.

6. A chart according to claim 1 or 2, **characterized by** the fact that the appearance characteristic other than color is non-uniformity of relief, at least two comparison samples having different distributions of relief.

7. A chart according to one of the preceding claims, **characterized by** the fact that, comprises at least three degrees of said appearance characteristic other than color, or indeed at least four, or even at least five.

8. A chart according to any one of preceding claims, **characterized by** the fact that the comparison samples present a non-uniform brightness.

9. The matching chart of claim 8, **characterized by** the fact that the shiniest regions are their brightness a gloss varnish (12).

10. The matching chart of precedent claim, **characterized by** the fact that the width (L) of the shiniest regions is approximately 300 micrometers.

11. The matching chart of any of the three immediately preceding claims, **characterized by** the fact that the dullest regions, are their matity to the presence of a mat varnish.

12. The matching chart of precedent claim, **characterized by** the fact that the width (d) of the dullest regions is approximately 100 micrometers.

13. The matching chart of any of preceding claims, **characterized by** the fact that the comparison samples (4) comprise a determined relief pattern, the relief pattern being configured to provide a non-uniform brightness.

14. The matching chart of precedent claim, **characterized by** the fact that the relief pattern comprises recesses and plateaus.

15. The matching chart according to precedent claim, **characterized by** the fact that the plateaus are coated with a gloss varnish, recesses being not coated with a gloss varnish.

16. A chart according to any one of claims 1 to 7, **characterized by** the fact that the comparison samples present substantially uniform brightness.

17. A chart according to any one of the preceding claims, **characterized by** the fact that each comparison sample (4) made on a medium that is generally rectangular in shape, preferably having dimensions of about 60 mm by 100 mm.

18. A chart according to any one of the preceding claims, **characterized by** the fact that each comparison sample (4) made on a medium that includes a hole (8), preferably situated about one-third of the way along its length, the hole preferably being circular with a diameter of about 20 mm.

19. The matching chart of any one of the preceding claims, **characterized by** the fact that each comparison sample includes an own identifier such as an alphanumeric code.

20. A method of manufacturing a cosmetic product, in particular a found makeup **characterized by** the fact that the method comprises the following steps:

  - providing a chart according to one of claims 1 to 19,
  - determining the comparison sample corresponding to the color and the appearance **characterized by** the fact that other than color of a person's skin or corresponding to color and a appearance characteristic other than the desired color.
  - making a cosmetic substance for application on a person's skin, in particular a found makeup, having the color and the appearance characteristic other than thus determined comparison sample color,

21. A method of determining the effectiveness of a cosmetic product, in particular a product that acts on the brightness of the skin, the method comprising the following steps:

  - determining which one of the comparison samples of the chart according to any one of claims 1 to 19 has the color and the appearance characteristic other than color, in particular brightness, that are closest to the color and the appearance characteristic other than color of the skin of a given person;
  - applying to the skin of said person the product;
  - observing any variation in the color and/or the appearance characteristic other than color, in particular brightness, of the skin by making a new comparison with the comparison samples of the chart.

22. A method of selecting a product intented to be applied on a keratinous element, **characterized by** the fact that it comprises the followings steps:

- providing a chart according as defined in any one of claims 1 to 19,
- selecting a comparison sample having a color and at least an appearance characteristic other than color that substantially correspond to the keratinous element so which the product is intented to be applied, and
- selecting a product from a plurality of different intented to be applied to the keratinous element, as a function of the comparison sample selected.

23. A method of treatment of a beratinous element, **characterized by** the fact that it comprises the followings steps:

- providing a matching chart as defined in any one of claims 1 to 19,
- selecting a comparison sample that corresponds to a desired color and at least desired appearance characteristic other than color of the beratinous element,
- treating said keratinous element based on said selected comparison sample.

24. A method of monitoring cosmetic treatment of a keratinous element with a product, **characterized by** the fact that the method comprising the following steps :

- providing a chart as defined in any one of claims 1 to 19,
- selecting a comparison sample that substantially corresponds to a color and at least one appearance characteristic other than color of the keratinous element,
- applying a product to the keratinous element, and
- determining whether the color and/or the at least one appearance characteristic other than color of the keratinous element to which the product has been applied has changed after applying the product by comparing the keratinous element with the comparison samples of the system.

**Patentansprüche**

1. Farbpalette, **dadurch gekennzeichnet, dass** sie mindestens zwei Vergleichsmuster umfasst, die ausgebildet sind, um das Aussehen eines Keratinelements zu simulieren, das aus der Haut, den Lippen, den Nägeln und den Haaren ausgewählt ist, wobei in der Farbpalette diese mindestens zwei Vergleichsmuster ausgebildet sind, um eine Farbe, die in dem kolorimetrischen Raum CIE L*C*h 1976 definiert ist, und in verschiedenen Graden mindestens ein anderes Aussehensmerkmal des Keratinelements als die Farbe zu simulieren.

2. Farbpalette nach dem vorhergehenden Anspruch, **dadurch gekennzeichnet, dass** jedes Vergleichsmuster (4) eine Farbe und ein anderes Aussehensmerkmal als die Farbe wiedergibt.

3. Farbpalette nach Anspruch 1 oder 2, **dadurch gekennzeichnet, dass** das andere Aussehensmerkmal als die Farbe der Glanz ist, wobei mindestens zwei Vergleichsmuster (4) vorzugsweise verschiedene Glanze haben.

4. Farbpalette nach Anspruch 1 oder 2, **dadurch gekennzeichnet, dass** das andere Aussehensmerkmal als die Farbe das Relief ist, wobei mindestens zwei Vergleichsmuster vorzugsweise verschiedene Reliefs haben.

5. Farbpalette nach dem vorhergehenden Anspruch, **dadurch gekennzeichnet, dass** sie mindestens zwei Vergleichsmuster umfasst, die Flecken einer sich von der Farbe des Hintergrunds unterscheidenden Farbe aufweisen, die in Anzahl und/oder in der Verteilung und/oder in der Größe von einem Muster zum anderen verschieden sind.

6. Farbpalette nach Anspruch 1 oder 2, **dadurch gekennzeichnet, dass** das andere Aussehensmerkmal als die Farbe die Reliefheterogenität ist, wobei mindestens zwei Vergleichsmuster verschiedene Verteilungen des Reliefs besitzen.

7. Farbpalette nach einem der vorhergehenden Ansprüche, **dadurch gekennzeichnet, dass** sie mindestens drei Grade dieses anderen Aussehensmerkmals als die Farbe besitzt, oder mindestens vier oder mindestens fünf.

8. Farbpalette nach einem der vorhergehenden Ansprüche, **dadurch gekennzeichnet, dass** die Vergleichsmuster einen nicht homogenen Glanz besitzen.

9. Farbpalette nach dem vorhergehenden Anspruch, **dadurch gekennzeichnet, dass** die glänzendsten Zonen ihren Glanz dem Vorhandensein eines glänzenden Lacks (12) verdanken.

10. Farbpalette nach dem vorhergehenden Anspruch, **dadurch gekennzeichnet, dass** die Breite (L) der glänzendsten Zonen nahe 300 μm ist.

11. Farbpalette nach einem der drei unmittelbar vorhergehenden Ansprüche, **dadurch gekennzeichnet, dass** die am wenigsten glänzenden Zonen ihre Mattheit dem Vorhandensein eines matten Lacks (13) verdanken.

12. Farbpalette nach dem vorhergehenden Anspruch, **dadurch gekennzeichnet, dass** die Breite (d) der am wenigsten glänzenden Zonen nahe 100 μm ist.

13. Farbpalette nach einem der vorhergehenden Ansprüche, **dadurch gekennzeichnet, dass** die Vergleichsmuster (4) ein bestimmtes Relief umfassen, das ihnen einen inhomogenen Glanz verleiht.

14. Farbpalette nach dem vorhergehenden Anspruch, **dadurch gekennzeichnet, dass** das Relief aus Vertiefungen und Plateaus besteht.

15. Farbpalette nach dem vorhergehenden Anspruch, **dadurch gekennzeichnet, dass** die Plateaus mit einem glänzenden Lack bedeckt sind, wobei die Vertiefungen diesen nicht aufweisen.

16. Farbpalette nach einem der Ansprüche 1 bis 7, **dadurch gekennzeichnet, dass** die Vergleichsmuster einen homogenen Glanz besitzen.

17. Farbpalette nach einem der vorhergehenden Ansprüche, **dadurch gekennzeichnet, dass** die Vergleichsmuster (4) jeweils auf einem Träger von rechteckiger allgemeiner Form und vorzugsweise mit Abmessungen nahe 60 mm auf 100 mm ausgeführt sind.

18. Farbpalette nach einem der vorhergehenden Ansprüche, **dadurch gekennzeichnet, dass** die Vergleichsmuster (4) jeweils auf einem Träger ausgeführt sind, der ein Loch (8) aufweist, das vorzugsweise auf etwa einem Drittel ihrer Länge gelegen ist, wobei dieses Loch vorzugsweise kreisförmig mit einem Durchmesser von nahe 20 mm ist.

19. Farbpalette nach einem der vorhergehenden Ansprüche, **dadurch gekennzeichnet, dass** jedes Vergleichsmuster eine eigene Kennung wie einen alphanumerischen Code umfasst.

20. Verfahren zur Herstellung eines kosmetischen Produkts, insbesondere einer Grundierung, **dadurch gekennzeichnet, dass** es die folgenden Schritte umfasst:

   - eine Farbpalette nach einem der Ansprüche bis 19 liefern,
   - das Vergleichsmuster bestimmen, das der Farbe und dem anderen Aussehensmerkmal als der Farbe der Haut einer Person entspricht oder einer Farbe und einem anderen Merkmal als der gewünschten Farbe entspricht,
   - ein kosmetisches Produkt, insbesondere eine Grundierung herstellen, die die Farbe und das andere Aussehensmerkmal als die Farbe des auf diese Weise bestimmten Vergleichsmusters besitzt, zum Zweck seines Auftrags auf die Haut dieser Person.

21. Verfahren zur Bestimmung der Wirksamkeit eines kosmetischen Produkts, insbesondere eines Produkts, das eine Wirkung auf den Glanz der Haut hat, **dadurch gekennzeichnet, dass** es die folgenden Schritte umfasst:

   - aus den Vergleichsmustern der Farbpalette nach einem der Ansprüche 1 bis 19 dasjenige bestimmen, dessen Farbe und dessen anderes Aussehensmerkmal als die Farbe, insbesondere der Glanz, denjenigen der Haut einer gegebenen Person so nahe wie möglich sind,
   - auf die Haut dieser Person das Produkt auftragen,
   - eine eventuelle Änderung der Farbe und/oder des anderen Aussehensmerkmals als der Farbe, insbesondere des Glanzes, der Haut suchen, indem ein neuer Vergleich mit den Vergleichsmustern der Farbpalette vorgenommen wird.

22. Verfahren zum Auswählen eines Produkts, das dazu bestimmt ist, auf ein Keratinelement aufgetragen zu werden, **dadurch gekennzeichnet, dass** es die folgenden Schritte umfasst:

   - eine Farbpalette, wie sie in einem der Ansprüche 1 bis 19 definiert ist, liefern,

- ein Vergleichsmuster auswählen, das eine Farbe und mindestens ein anderes Aussehensmerkmal als die Farbe besitzt, die bzw. das im Wesentlichen dem Keratinelement entspricht, auf das das Produkt aufgetragen werden soll, und
- aus einer Vielzahl von verschiedenen Produkten, die dazu bestimmt sind, auf das Keratinelement aufgetragen zu werden, in Abhängigkeit von dem ausgewählten Vergleichsmuster ein Produkt auswählen.

23. Verfahren zur kosmetischen Behandlung eines Keratinelements, **dadurch gekennzeichnet, dass** es die folgenden Schritte umfasst:

- eine Farbpalette, wie sie in einem der Ansprüche 1 bis 19 definiert ist, liefern,
- ein Vergleichsmuster auswählen, das einer gewünschten Farbe und mindestens einem gewünschten anderen Aussehensmerkmal als der Farbe des Keratinelements entspricht,
- das Keratinelement in Abhängigkeit von dem ausgewählten Vergleichsmuster behandeln.

24. Verfahren zur Verfolgung der kosmetischen Behandlung eines Keratinelements mit einem Produkt, **dadurch gekennzeichnet, dass** es die folgenden Schritte umfasst:

- eine Farbpalette, wie sie in einem der Ansprüche 1 bis 19 definiert ist, liefern,
- ein Vergleichsmuster auswählen, das im Wesentlichen einer Farbe und mindestens einem anderen Aussehensmerkmal als der Farbe des Keratinelements entspricht,
- das Produkt auf das Keratinelement auftragen und
- bestimmen; ob die Farbe und/oder das mindestens eine andere Aussehensmerkmal als die Farbe des Keratinelements, auf das das Produkt aufgetragen wurde, sich nach Auftragen des Produkts geändert hat, indem das Keratinelement und die Vergleichsmuster des Systems verglichen werden.

FIG.1

FIG.2

FIG.3

FIG_4

FIG.5

11  10  11  10

4

FIG_6

12  12  12  12

4

FIG_7

13  13  13

4

FIG_8

12  13  12  13  12  13  12

4

FIG_9

12

L  d

12

FIG_12

FIG.10

FIG.11